(19) Europäisches Patentamt
European Patent Office
Office européen des brevets

(11) EP 0 840 119 B1

(12) **FASCICULE DE BREVET EUROPEEN**

(45) Date de publication et mention
de la délivrance du brevet:
**18.06.2003 Bulletin 2003/25**

(51) Int Cl.$^7$: **G01N 33/00**

(21) Numéro de dépôt: **97402404.4**

(22) Date de dépôt: **13.10.1997**

(54) **Procédé et dispositif pour fournir un gaz pure à un appareil, ledit gaz étant chargé d'une quantité prédeterminée d'au moins une impureté gazeouse**

Verfahren und Vorrichtung zum Liefern eines reinen Gases an eine Vorrichtung, wobei das Gas eine vorbestimmte Menge an mindenstens einer gasförmigen Verunreinigung enthält

Method and apparatus for supplying a pure gas to an apparatus, said gas being charged with a predetermined quantity of at least one gaseous impurity

(84) Etats contractants désignés:
**BE DE FR GB IT NL SE**

(30) Priorité: **05.11.1996 FR 9613436**

(43) Date de publication de la demande:
**06.05.1998 Bulletin 1998/19**

(73) Titulaire: **L'air Liquide, S.A. à Directoire et Conseil de Surveillance pour l'Etude et l'Exploitation des Procédés Georges Claude**
**75321 Paris Cedex 07 (FR)**

(72) Inventeurs:
• **Girard, Jean-Marc**
**75010 Paris (FR)**
• **Mail, Alain**
**38420 Domene (FR)**
• **Marot, Yves**
**Rue Huguier, 78530 Buc (FR)**

(74) Mandataire: **Vesin, Jacques et al**
**L'AIR LIQUIDE, S.A.,**
**Service Propriété Industrielle,**
**75, Quai d'Orsay**
**75321 Paris Cédex 07 (FR)**

(56) Documents cités:
**EP-A- 0 528 386      EP-A- 0 664 449**
**US-A- 5 239 856**

• **K. SIEFERING ET AL.: "Quantitative analysis of contaminants in ultrapure gases at the parts-per-trillion level using atmospheric pressure ionization mass spectroscopy" JOURNAL OF VACUUM SCIENCE AND TECHNOLOGY: PART A, vol. 11, no. 4, juillet 1993 - août 1993, NEW YORK US, pages 1593-1597, XP000403759**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**Description**

L'invention est relative à un procédé et à un dispositif pour fournir à un appareil, notamment un analyseur de traces d'impuretés tel qu'un spectromètre de masse à ionisation à pression atmosphérique, un gaz pur chargé d'une quantité prédéterminée d'au moins une impureté gazeuse.

Dans le domaine de l'analyse des gaz de très haute pureté, on utilise souvent des analyseurs conçus pour détecter des impuretés à de très faibles concentrations ($10^{-2}$ à $10^{-5}$ppm par exemple, voire $10^{-3}$ à $10^{-6}$ppm). Un tel appareil nécessite, pour assurer un fonctionnement fiable et précis, un étalonnage à des intervalles de temps réguliers.

Un tel étalonnage est effectué en fournissant séquentiellement à l'analyseur d'abord un gaz pur ou "zéro", c'est-à-dire un gaz contenant moins de $10^{-5}$ ppm d'impuretés, puis un gaz dit "d'étalonnage" contenant des impuretés à des concentrations précisément connues.

On connaît, du document FR-A-2714968 au nom de la Demanderesse et du document US-A-5 239 856 également au nom de la Demanderesse, de tels procédés et de tels dispositifs conçus pour l'alimentation d'un analyseur à très haute sensibilité.

Le document FR précité décrit un dispositif et un procédé dans lesquels un gaz pur est chargé de traces d'impuretés à l'aide d'une batterie de cartouches de perméation raccordées en parallèle à une unité de dilution des impuretés dans le gaz pur. Chaque cartouche contient une impureté gazeuse telle que par exemple $H_2O$, $CO_2$, CO, $O_2$, $CH_4$, $H_2$, etc..., qui diffuse en quantité très faible à travers une membrane pour charger le gaz pur circulant dans l'unité de dilution.

Ce dispositif présente l'inconvénient que chaque cartouche de perméation crée, par sa ligne de raccordement à l'unité de dilution, une branche dans laquelle l'écoulement stagne, ce qui provoque des interactions non désirées avec les parois de la ligne, telles que par exemple des phénomènes d'absorption et de désorption.

De plus, ces branches de raccordement sont difficiles à purger, ce qui compromet l'utilisation d'une telle batterie de cartouches de perméation avec différentes sortes de gaz purs.

Par ailleurs, le débit de l'impureté introduite par une cartouche de perméation varie dans le temps. Par conséquent, la précision avec laquelle la composition du gaz d'étalonnage est connue, diminue avec le temps.

Un autre inconvénient du dispositif est lié au fonctionnement des cartouches de perméation. Une cartouche de perméation est réalisée par un réservoir comportant une sortie obturée par une membrane, par exemple en silicone, à travers laquelle diffuse l'impureté contenue dans le réservoir.

Cependant, cette membrane n'est pas seulement perméable dans le sens cartouche-unité de dilution, mais aussi dans le sens inverse unité de dilution-cartouche.

Ainsi, le gaz pur se trouvant dans la branche de raccordement peut diffuser à travers la membrane dans la cartouche, notamment quand la molécule est petite telle que la molécule $H_2$. Au cours du temps, il se crée une composition inconnue de gaz dans la cartouche de perméation, ce qui compromet, par conséquent, la génération précise d'un gaz d'étalonnage, notamment dans le cas de l'utilisation séquentielle du dispositif avec différentes sortes de gaz purs.

On procède aussi couramment de la façon suivante : en disposant de mélanges gazeux preeffectués gaz pur/impureté I, à teneur que l'on peut qualifier d'intermédiaire, par exemple de l'ordre de grandeur du ppm.

On dispose ainsi par exemple de bouteilles premélangées comportant quelques dizaines de ppm de CO ou encore d'hydrogène dans l'azote.

On effectue alors les étapes de dilutions requises, à l'aide du gaz pur correspondant (Ar, $N_2$, He...), du gaz de chaque bouteille de premélange, pour parvenir au mélange final comportant l'impureté I dans le gaz pur considéré à une teneur par exemple de l'ordre de grandeur du ppb.

On conçoit alors ce qu'une telle procédure représente de complexité, de lourdeur, et de multiplication des sources d'erreurs et de pollution des mélanges au différents stades de la fabrication.

Le document US-A-5 239 856 décrit pour sa part un procédé et un dispositif de fabrication d'un gaz pur chargé de traces d'impuretés, suivant lesquels on prélève une certaine quantité de gaz à partir de deux sources de gaz dont chaque gaz constitue une impureté dans un gaz pur.

On mélange les gaz prélevés, et on dilue le mélange dans une unité de dilution afin d'obtenir une très faible concentration des impuretés dans le gaz pur, concentration située dans une plage s'étendant de $10^{-5}$ppm à $10^{-2}$ppm, afin de fournir ce gaz d'étalonnage à un analyseur de traces d'impuretés qui doit être calibré.

La précision avec laquelle les concentrations des traces d'impuretés dans le gaz pur sont connues dépend non seulement de la qualité de l'unité de dilution, mais aussi de la qualité de fabrication du mélange de gaz. Or, dans le cas du US-A précité, cette précision dépend de l'incertitude de réglage des différents débitmètres massiques disposés dans les lignes de prélèvement des impuretés. En augmentant le nombre de sources d'impureté et, par conséquent le nombre de débitmètres massiques pour fabriquer le mélange, on diminue la précision avec laquelle la composition du mélange de gaz est connue, puisque les incertitudes de débit de chaque débitmètre massique s'additionnent pour constituer l'incertitude sur la composition du mélange de gaz obtenu.

On pourra également se reporter au document EP-A-528 386 qui illustre l'Etat de la Technique de la fabrication de mélanges de calibration d'un analyseur de gaz.

La présente invention vise à pallier ces divers inconvénients en proposant un procédé et un dispositif de fourniture à un appareil d'un gaz pur chargé d'une quantité prédéterminée d'au moins une impureté gazeuse, qui soit fiable, précis, et facile à mettre en oeuvre.

A cet effet, l'invention a pour objet un procédé de fourniture à un appareil d'un gaz pur chargé d'une quantité prédéterminée d'au moins une impureté gazeuse, suivant lequel on mélange préalablement au moins deux gaz auxiliaires différents dont ladite impureté, on dilue de façon prédéterminée une quantité prélevée dudit mélange dans le gaz pur, et on fournit le gaz pur chargé de ladite impureté à l'appareil, se caractérisant en ce qu'on stocke lesdits gaz auxiliaires sous forme d'un mélange de composition connue, dans un réservoir, les concentrations volumiques de la majorité des gaz auxiliaires dudit mélange étant sensiblement du même ordre de grandeur, et on prélève le mélange de ce réservoir pour effectuer la dilution dans le gaz pur.

Selon une des mises en oeuvre de l'invention, le mélange comprend au moins un gaz inerte dont la concentration volumique est supérieure à la concentration volumique de chacun des autres gaz du mélange.

La concentration volumique du gaz inerte est alors avantageusement supérieure à la somme des concentrations volumiques des autres gaz du mélange.

Selon une mise en oeuvre avantageuse de l'invention, l' « ordre de grandeur » des teneurs est le %.

Selon la composition du mélange (en particulier selon les espèces de calibration recherchées), on notera que pour des raisons de sécurité, il est avantageux de disposer d'un ou plusieurs gaz inertes dans le mélange, et de choisir une concentration du gaz plus élevée que la concentration volumique de tous les autres constituants du mélange. Grâce à une telle composition de mélange, des oxydants, tels que $O_2$ ou CO, peuvent coexister ensemble avec des combustibles, tels que par exemple $CH_4$, sans qu'il y ait un risque d'inflammabilité ou d'explosion du réservoir contenant le mélange.

On peut ainsi, à titre illustratif, adopter pour tous les constituants non inertes du mélange une teneur voisine du % ou de quelques %, mais adopter pour le ou les gaz inertes du mélange une teneur de quelques dizaines de %, sans pour cela sortir du cadre de la présente invention, puisque les teneurs de tous les composants du mélange s'exprime selon le même ordre de grandeur (%).

L'invention a également pour objet un dispositif pour la mise en oeuvre du procédé défini ci-dessus, comprenant une source de gaz pur, une source de ladite impureté gazeuse, et des moyens de dilution prédéterminée de l'impureté dans le gaz pur, reliés à l'appareil par l'intermédiaire d'une ligne de fourniture de gaz, caractérisé en ce que ladite source d'impureté comprend un réservoir contenant ledit mélange de composition connue d'au moins deux gaz différents dont ladite impureté, les concentrations volumiques de la majorité des gaz du mélange étant sensiblement du même ordre de grandeur.

Le dispositif suivant l'invention peut comporter une plusieurs des caractéristiques suivantes :

- les moyens de dilution comprennent des moyens de division du débit de ladite source de gaz pur, et plusieurs étages de dilution disposés en série, chaque étage comprenant une première ligne d'entrée reliée à une ligne de sortie associée desdits moyens de division de débit et une deuxième ligne d'entrée reliée à une ligne de sortie respective de l'étage de dilution disposé juste en amont, la deuxième ligne d'entrée du premier étage de dilution étant reliée audit réservoir par l'intermédiaire d'une ligne de prélèvement dudit mélange,
- la deuxième ligne d'entrée d'au moins un étage de dilution comporte un organe additionnel de régulation de débit,
- chaque étage de dilution comprend des moyens de mélange des gaz alimentant les entrées de l'étage et au moins un étage de dilution comprenant une ligne de purge associée, reliée par une extrémité audits moyens de mélange et dans laquelle est disposé un déverseur pour régler la pression dans l'étage de dilution associé,
- un étage de dilution comprend une troisième ligne d'entrée reliée à des moyens pour charger le gaz pur d'une quantité prédéterminée de $H_2O$,
- les moyens de division du débit de ladite source comprenant une ligne principale de fourniture de gaz pur reliée à plusieurs lignes de sortie secondaires, disposées en parallèle, et comportant chacune une restriction calibrée,
- la source de gaz pur comprend une source de gaz à analyser et une unité d'épuration du gaz débité par la source,
- l'unité d'épuration comprend au moins deux épurateurs, l'entrée de chaque épurateur étant reliée à une vanne de branchement, disposée dans une ligne d'entrée de l'unité d'épuration, et la sortie de chaque épurateur étant reliée à une vanne de mise en circuit du gaz épuré, disposée dans une ligne d'alimentation des moyens de dilution,
- la ligne d'entrée de l'unité d'épuration et la ligne d'alimentation des moyens de dilution débouchent chacune par une extrémité dans une ligne de purge correspondante, un organe de création d'une perte de charge étant disposé dans chaque ligne de purge correspondante,
- chaque vanne de branchement ainsi que chaque vanne de mise en circuit comprend un premier conduit relié en permanence par une première extrémité à un épurateur associé, un deuxième conduit, le deuxième conduit des vannes de branchement étant disposé dans la ligne d'entrée de l'unité d'épuration et ceux de la vanne de mise en circuit étant disposé dans la ligne d'alimentation des moyens de dilution, et un actionneur commutable entre une position de mise en communication du premier conduit avec le deuxième conduit et une position d'isolement du premier conduit par rapport au deuxième conduit, le deuxième conduit étant libre de volumes de stagnation d'écoulement,

- le deuxième conduit de chaque vanne comporte une chambre dans laquelle débouche la deuxième extrémité du premier conduit, et chaque vanne comporte un organe d'obturation, sur lequel agit l'actionneur de la vanne, qui obture, dans ladite position d'isolement, l'extrémité du premier conduit débouchant dans ladite chambre, et qui est en retrait par rapport à cette extrémité du premier conduit dans ladite position de mise en communication,

- l'extrémité du premier conduit débouchant dans ladite chambre est munie d'un joint d'étanchéité en saillie dans la chambre, et l'organe d'obturation comprend une membrane élastiquement déformable formant une partie de paroi de la chambre opposée au joint d'étanchéité, la membrane étant appliquée de façon étanche sur le joint d'étanchéité à l'encontre de la force d'élasticité de la membrane, dans ladite position d'isolement, par un poussoir de l'actionneur,

- chaque vanne comporte des moyens de commande de la commutation de l'actionneur entre lesdites positions de mise en communication et d'isolement, les moyens de commande de chaque vanne sont reliés à une unité de commande d'isolement ou de mise en communication de chaque vanne, l'unité de commande comporte des moyens logiques qui interdisent la commutation simultanée d'actionneurs associés à des épurateurs différents en position de communication.

De plus, l'invention a pour objet un réservoir contenant un mélange de gaz de composition étalonnée pour charger un gaz pur d'impuretés suivant le procédé défini ci-dessus et/ou pour constituer la source d'impuretés du dispositif défini ci-dessus.

Suivant une autre caractéristique, le réservoir contient au moins deux gaz différents à des teneurs sensiblement du même ordre de grandeur, et un gaz inerte en proportion plus importante et, de préférence, supérieure à la somme des proportions des autres gaz.

En outre, l'invention a pour objet une vanne pour le dispositif défini ci-dessus.

D'autres caractéristiques et avantages de l'invention ressortiront de la description suivante donnée à titre d'exemple sans caractère limitatif, en regard des dessins annexés sur lesquels :

La figure 1 est un schéma général d'une installation pour fournir à un appareil, soit un gaz à analyser, soit un gaz pur, ou encore un gaz chargé d'une quantité prédéterminée d'impuretés ;

La figure 2 est une vue en coupe suivant la ligne II/II de la figure 3 d'une vanne du dispositif de régulation de la figure 1 ainsi que d'une vanne d'une unité d'épuration de la figure 1, en position d'isolement;

La figure 3 est une vue en coupe, suivant la ligne III/III de la figure 2, de la même vanne en position de mise en communication;

La figure 4A est une vue en coupe, suivant la ligne IV/IV de la figure 2, de la vanne en position d'isolement;

La figure 4B est une vue en coupe, suivant la ligne IV/IV de la figure 2, de la vanne en position de mise en communication; et

La figure 5 est un schéma d'un étage de dilution.

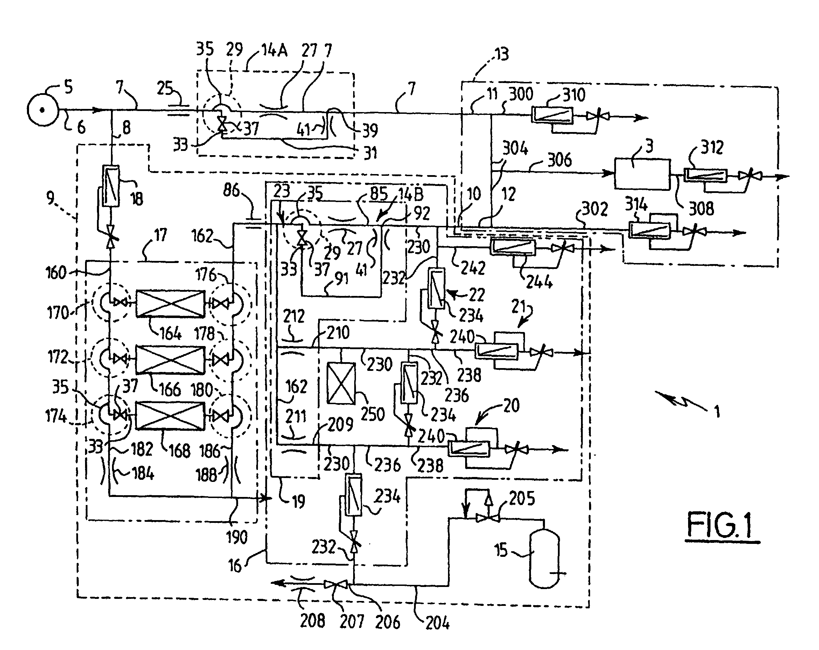

La figure 1 montre une installation 1 de fourniture de gaz à un appareil 3 tel que par exemple un analyseur de traces d'impuretés dans un gaz du type spectromètre de masse à ionisation à pression atmosphérique. Un tel analyseur 3 est capable de mesurer des traces d'impuretés dans un gaz à des concentrations très faibles comprises entre $10^{-2}$ à $10^{-5}$ppm, voire $10^{-3}$ à $10^{-6}$ppm. Comme on va l'expliquer en détail ci-après, cette installation 1 permet de fournir à l'appareil 3 alternativement un gaz pur de référence ou "gaz zéro", c'est-à-dire un gaz contenant typiquement moins de $10^{-4}$ ppm d'impuretés, un gaz chargé d'une quantité prédéterminée d'impuretés gazeuses connues telles que par exemple $H_2O$, $CO_2$, $CO$, $O_2$, $CH_4$, $H_2$, etc..., à des concentrations variables dans une plage s'étendant de $10^{-5}$ppm à $10^{-2}$ppm, ou encore un gaz à analyser. En outre, cette installation doit permettre de contrôler les paramètres d'introduction du gaz dans l'analyseur 3, tels que la pression et le débit.

De plus, cette installation doit permettre l'utilisation successive de différentes sortes de gaz à analyser.

A cet effet, une source 5 d'un gaz sous pression à analyser est reliée par l'intermédiaire d'une ligne de prélèvement 6 à l'installation de fourniture de gaz 1. Cette source 5 comprend par exemple une source unique d'une sorte de gaz à analyser, ou plusieurs sources de pression de différentes sortes de gaz reliées chacune par une ligne de prélèvement à un dispositif destiné à fournir l'un quelconque de plusieurs gaz à un appareil, tel que le dispositif décrit dans la demande de brevet français R-2749924 déposée au nom de la Demanderesse.

La ligne de prélèvement 6 est reliée d'une part à une ligne d'analyse 7 et d'autre part à une ligne d'alimentation 8 d'un dispositif 9 de fourniture d'un gaz pur ou charge d'une quantité prédéterminée d'impuretés gazeuses.

La ligne d'analyse 7 et une ligne de sortie 10 du dispositif de fourniture 9 sont reliées chacune à une ligne d'entrée correspondante 11, 12 d'un dispositif de sélection 13 pour fournir à l'appareil 3 soit le gaz contenu dans la ligne d'analyse 7, soit le gaz débité par la ligne de sortie 10 du dispositif de fourniture 9.

Un dispositif de régulation 14A d'un débit prédéterminé de gaz pour des gaz à analyser ayant des masses molaires sensiblement différentes, est disposé dans la ligne d'analyse 7.

Le dispositif de fourniture 9 comprend une source de gaz pur, une source d'impuretés 15, et des moyens 16 de dilution prédéterminée des impuretés dans le gaz pur.

La source de gaz pur est constituée d'une part par la source 5 du gaz à analyser et, d'autre part, par une unité d'épuration 17 du gaz débité par la source 5, le débit de gaz alimentant l'unité d'épuration étant réglé par l'intermédiaire d'un régulateur de débit massique 18 disposé dans la ligne d'alimentation 8.

Les moyens de dilution 16 comportent des moyens 19 de division du débit de gaz en sortie de l'unité d'épuration 17, qui alimentent plusieurs étages de dilution 20, 21, 22 disposés en série.

Une branche 23 des moyens 19 de division du débit comporte un dispositif 14B de régulation d'une pression amont prédéterminée de gaz pour des gaz ayant des masses molaires sensiblement différentes, la structure de ce dispositif 14B étant identique à celle du dispositif de régulation 14A.

On va décrire ci-après en détail la structure et le fonctionnement des différentes unités de l'installation de fourniture de gaz 1.

I. Dispositif de régulation de l'écoulement de gaz ayant des masses molaires sensiblement différentes.

I.1. Structure du dispositif de régulation -

Le dispositif de régulation 14A est disposé dans la ligne d'analyse 7. En amont de ce dispositif de régulation 14A, est disposée une jauge de pression 25 pour déterminer la pression en amont du dispositif de régulation 14A.

Le dispositif de régulation 14A comporte, pour le mode de réalisation représenté, une restriction calibrée 27, par exemple un orifice calibré, disposée dans la ligne d'analyse 7. En amont de l'orifice 27 est disposée une vanne de branchement 29, représentée de façon schématique entourée par des tirets, pour la mise en service sélective d'une ligne de dérivation 31.

La vanne 29 comprend un premier conduit 33 relié en permanence par une extrémité à la ligne de dérivation 31. Elle comprend en outre un deuxième conduit 35 toujours ouvert qui est disposé dans la ligne d'analyse 7.

Le premier conduit 33 et le deuxième conduit 35 de la vanne 29 peuvent être mis en communication par l'intermédiaire d'un actionneur 37, comme cela sera expliqué en détail ci-après, commutable entre une position de mise en communication du premier conduit 33 avec le deuxième conduit 35, et une position d'isolement du premier conduit 33 par rapport au deuxième conduit 35. La ligne de dérivation 31 est reliée par son autre extrémité 39, en aval de l'orifice 27, à la ligne d'analyse 7.

Un deuxième orifice calibré 41 est avantageusement disposé dans la ligne de dérivation 31, au plus près de l'extrémité 39 de celle-ci. Ainsi, un volume de stagnation d'écoulement, formé en position d'isolement des deux conduits 33 et 35 par la partie de la ligne de dérivation 31 située entre l'orifice 41 et l'extrémité 39, est le plus petit possible.

La structure du dispositif de régulation 14B est ici identique à celle du dispositif 14A. C'est pourquoi les éléments identiques portent les mêmes numéros de référence.

Ainsi, ce dispositif comporte un premier orifice 27 disposé dans une ligne 85 d'alimentation d'un gaz pur débité en sortie de l'unité d'épuration 17. Dans cette ligne 85 est disposée une vanne de branchement 29 identique à celle du dispositif 14A. Au conduit 33 de la vanne 29 est reliée une extrémité d'une ligne de dérivation 91. L'autre extrémité de cette ligne 91 est raccordée en aval de l'orifice 27 à la ligne d'alimentation 85. Un orifice calibré 41 est disposé au plus près de l'extrémité 92 par laquelle la ligne de dérivation 91 est raccordée à la ligne d'alimentation 85.

I.2. Structure de la vanne des dispositifs de régulation :

On décrit ci-après en détail un exemple de réalisation de la vanne 29 installée dans les dispositifs de régulation 14A et 14B. Une telle vanne, du type DAD électropolie, est par exemple dérivée d'une vanne commercialisée par la société NUPRO et fabriquée par la société SWAGELOK.

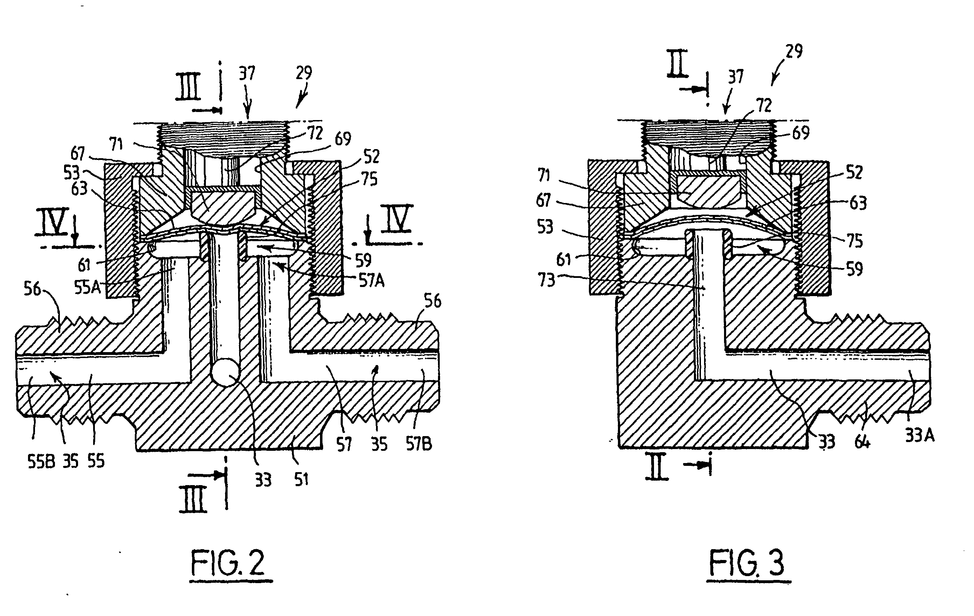

Comme cela est représenté sur les figures 2 et 3, la vanne 29 comporte un corps 51 dans lequel sont réalisés le premier conduit 33 et le deuxième conduit 35, un obturateur 52 et un actionneur 37, représenté en partie, vissé sur le corps 51 au moyen d'un écrou 53.

Le deuxième conduit 35 (Fig.2) est formé par deux tronçons de conduit 55 et 57 et par une chambre annulaire 59, à symétrie de révolution. Dans une partie latérale du fond de cette chambre 59 débouche l'une 55A, 57A des deux extrémités de chaque tronçon de conduit 55, 57.

L'autre extrémité 55B, 57B de chaque tronçon de conduit 55, 57 débouche dans un raccord respectif latéral 56 du corps 51. Ces extrémités 55B et 57B sont diamétralement opposées. Les deux raccords 56 sont destinés à être reliés à la ligne d'analyse 7 en ce qui concerne le dispositif de régulation 14A et à ligne d'alimentation 85 en ce qui concerne le dispositif de régulation 14B.

La chambre 59 est formée par un évidement 61 sensiblement cylindrique, ménagé dans la face supérieure du corps 51, et par l'obturateur 52. Cet obturateur est lui-même constitué d'un ensemble de deux membranes accolées 63 qui recouvrent l'évidement 61 et constituent la paroi supérieure de la chambre 59.

Les membranes 63 sont réalisées en un matériau élastiquement déformable, par exemple du métal. Chaque membrane 63 est un disque dont la partie centrale est bombée dans une direction opposée du corps 51. Le bord des membranes 63 est serré de façon étanche entre le bord annulaire de l'évidement 61 et un bord annulaire d'une pièce de maintien 67 qui fait partie de l'actionneur 37. La pièce 67 est réalisée en forme de cuvette afin de permettre un débattement de la partie bombée des membranes 63.

La pièce de maintien 67 comporte dans sa partie centrale, en regard des membranes 63, un alésage 69 de guidage dans lequel peut coulisser un poussoir 71 mû par une tige 72 de l'actionneur 37.

Le premier conduit 33 de la vanne 29 comprend un simple perçage borgne droit qui s'étend perpendiculairement à l'axe défini par les extrémités 55B, 57B des tronçons de conduit 55, 57 et une cheminée de raccordement 73 qui débouche au centre de l'évidement 61.

Une extrémité 33A, du premier conduit 33 débouche dans un raccord latéral respectif 64 du corps 51, et est destinée à être reliée à la ligne de dérivation 31 dans le cas du dispositif 14A ou à la ligne de dérivation 91 dans le cas du dispositif 14B.

L'extrémité de la cheminée 73 débouchant dans l'évidement 61 comporte un joint d'étanchéité cylindrique 75 emmanché à force dans le corps 51 de la vanne et qui fait saillie dans la chambre 59.

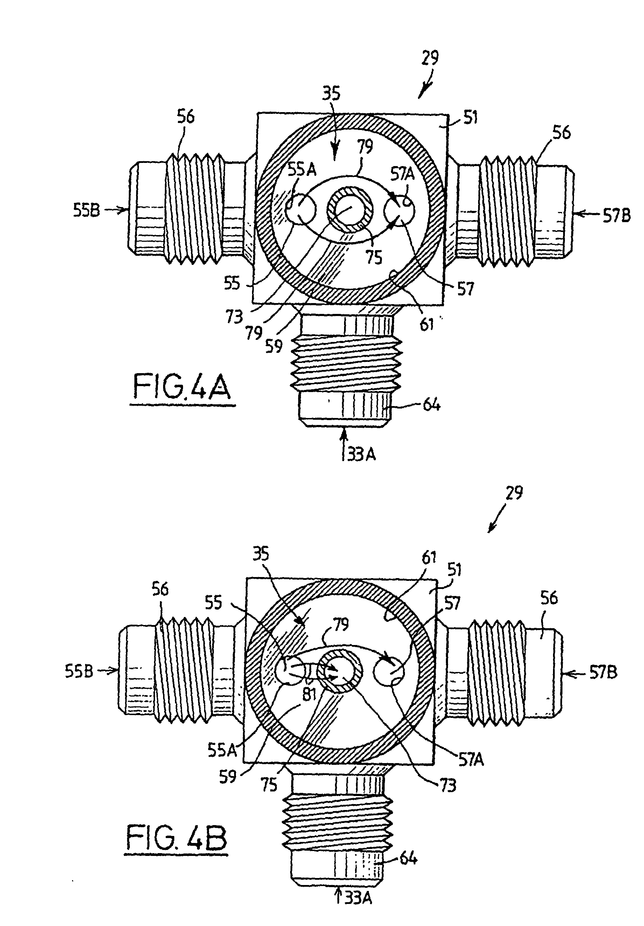

Les figures 2 et 4A montrent la vanne 29 en position d'isolement du premier conduit 33 par rapport au deuxième conduit 35. Dans un tel cas, la partie centrale des membranes 63 est pressée de façon étanche par le poussoir 71 sur le joint d'étanchéité 75, de sorte que la cheminée 73 est isolée par rapport à la chambre 59.

Un gaz introduit dans le deuxième conduit 35 de la vanne circule néanmoins librement, par exemple du tronçon de conduit 55 dans la chambre 59 et, ensuite, dans le tronçon de conduit 57 comme cela est représenté par les flèches 79 sur la figure 4A. On comprend que le deuxième conduit 35 de la vanne ainsi formée ne présente aucun volume de stagnation d'écoulement.

La figure 3 et la figure 4B correspondent à une position de mise en communication du premier conduit 33 avec le deuxième conduit 35. Dans un tel cas, le poussoir 71 est en retrait. Par la force d'élasticité des membranes 63, celles-ci reprennent leur forme initiale bombée. Par conséquent, il se forme un espace libre entre les membranes 63 et le joint d'étanchéité 75, de sorte que le gaz s'écoulant dans le deuxième conduit 35 se déverse en grande partie via la cheminée 73 dans le premier conduit 33 comme cela est représenté par les flèches 81 sur la figure 4B.

I.3. Fonctionnement du dispositif de régulation :

On explique ci-après le fonctionnement des dispositifs de régulation 14A, 14B en regard de la figure 1. A cet effet, on distingue deux modes de fonctionnement.

Dans un premier mode de fonctionnement, la pression en amont du dispositif de régulation est imposée, et le débit doit être à peu près le même pour deux gaz ayant des masses molaires sensiblement différentes. Ce mode de fonctionnement correspond au dispositif 14A disposé dans la ligne d'analyse 7.

Dans un deuxième mode de fonctionnement, le débit de gaz en amont du dispositif de régulation est imposé, et on veut assurer un régime d'écoulement sonique. Ce mode de fonctionnement correspond à celui qui est réalisé par le dispositif de régulation 14B.

I.3.1. Fonctionnement à pression imposée :

Pour un gaz léger, tel que par exemple l'hydrogène, on commute la vanne 29 du dispositif de régulation 14A dans la position d'isolement du premier conduit 33 par rapport au deuxième conduit 35. Le gaz de la source 5 s'écoule librement dans la ligne d'analyse 7 à travers le deuxième conduit 35 et l'orifice 27. Pour un écoulement en régime sonique, c'est-à-dire dans le cas où le rapport entre la pression amont de l'orifice 27 et la pression aval de cet orifice est supérieur à 2, on sait que le débit volumique $D_{27}$ du gaz à travers l'orifice 27 est égale à :

$$D27 = K \times P \times S_{27} \times M^{-1/2}$$

avec :

P = pression en amont de l'orifice 27,

$S_{27}$ = section de l'orifice 27,
M = masse molaire du gaz s'écoulant à travers l'orifice, et
K = constante qui dépend de la température et de la nature des gaz.

Grâce au fait que le conduit 35 de la vanne 29 ne présente pas de volume de stagnation d'écoulement et que l'orifice 41 de la ligne de dérivation 31 est disposé près de l'extrémité 39 de cette ligne, le dispositif de régulation 14A n'introduit qu'un volume de stagnation d'écoulement négligeable dans la ligne d'analyse 7 en position d'isolement du premier conduit 33 par rapport au deuxième conduit 35 de la vanne.

Dans le cas d'un gaz, tel que par exemple $N_2$, ayant une masse molaire plus importante, à pression égale, le débit à travers l'orifice 27 chute proportionnellement à la racine carrée du rapport des masses molaires des deux gaz, par exemple pour $N_2$ et $H_2$, à environ un quart du débit obtenu pour $H_2$. Pour maintenir dans ce cas le débit fourni par la ligne d'analyse 7 à peu près au même niveau que le débit d'un gaz de masse molaire faible, la vanne 29 est commutée à l'état de mise en communication du premier conduit 33 avec le deuxième conduit 35. Le gaz s'écoule alors non seulement à travers l'orifice 27, mais aussi dans la ligne de dérivation 31 à travers l'orifice 41.

Au débit $D_{27}$ s'ajoute alors, au raccordement des lignes d'analyse 7 et de dérivation 31, le débit $D_{41}$ qui est donné, en régime sonique, par la relation :

$$D_{41} = K \times P \times S_{41} \times M^{-1/2}.$$

La nomenclature utilisée est analogue à celle utilisée pour le débit $D_{27}$.

Afin que le débit régulé par le dispositif de régulation 14A soit à peu près égal pour deux gaz ayant respectivement une masse molaire $M_1$ et $M_2$, on choisit la section $S_{41}$ de l'orifice 41 de telle sorte qu'elle satisfait la relation :

$$S_{41} = ((M_1^{1/2} / M_2^{1/2}) \times S_{27}) - S_{27}$$

où $M_1$ est la masse molaire d'un gaz ayant une masse molaire importante, et
$M_2$ est la masse molaire d'un gaz ayant une masse molaire faible.

De préférence, on choisit la section $S_{41}$ de l'orifice 41 de telle sorte que le débit de $H_2$ à l'état d'isolement de la vanne 29 est comparable au débit de $N_2$ à travers l'orifice 27 et l'orifice 41 à l'état de mise en communication de la vanne 29.

I.3.2. Fonctionnement à débit imposé :

Ce mode de fonctionnement du dispositif de régulation est intéressant dans le cas où le débit en amont du dispositif de régulation est imposé, et où il faut assurer un régime sonique pour des orifices de régulation de débit, tels que par exemple ceux des moyens de division de débit 19. Le débit de gaz en amont du dispositif de régulation 14B est imposé par le régulateur de débit 18.

On comprendra ici que le régime « sonique » est préféré et que le fait de s'en éloigner trop rend tout simplement difficile le calcul de la répartition des débits. On s'attachera alors à rechercher un rapport des débits amont et aval se situant entre 1,5 et 30.

Pour expliquer le fonctionnement à débit imposé, on ne considère que le dispositif de régulation 14B seul disposé dans la ligne d'alimentation 85, comme cela a été décrit au sujet du dispositif 14A. Dans le cas où le dispositif 14B est disposé dans les moyens 19 de division de débit, le raisonnement présenté ci-après s'applique de manière correspondante.

A débit volumique imposé, la pression en amont de l'orifice 27 en position d'isolement de la vanne 29 est donnée par la relation :

$$P = K' \times D_{18} \times M^{1/2} \times S_{83}^{-1}$$

avec :

$D_{18}$ = débit volumique imposé par le régulateur de débit 18,
K' = une constante qui dépend de la nature du gaz et de la température,
$S_{27}$ = la section de l'orifice 27.

La section de l'orifice 27 est dimensionnée de telle manière que pour un gaz léger tel que $H_2$, on atteint des conditions soniques en amont de l'orifice 27.

Dans le cas d'un gaz, tel que $N_2$, ayant une masse molaire importante, à débit égal, la pression en amont de l'orifice 27 est environ quatre fois supérieure par rapport à la pression amont obtenue pour $H_2$.

C'est pourquoi on commute la vanne 29 à l'état de mise en communication du premier conduit 33 avec le deuxième conduit 35. Le gaz s'écoule alors non seulement à travers l'orifice 27, mais aussi à travers l'orifice 41. La pression en amont de l'orifice 27 ainsi que de l'orifice 41 est donnée par la relation

$$P = K' \times D_{18} \times M^{1/2} \times (S_{27} + S_{41})^{-1}$$

avec :

$S_{41}$ = section de l'orifice 41.

Pour que la pression en amont du dispositif de régulation 14B atteigne les conditions soniques pour deux gaz de masse molaire différente, on choisit la section $S_{41}$ de l'orifice 41 de telle sorte qu'elle vérifie la relation

$$S_{41} = ((M_1^{1/2} / M_2^{1/2}) \times S_{27}) - S_{27}$$

où
$M_1$ est la masse molaire d'un gaz de masse molaire importante, et $M_2$ est la masse molaire d'un gaz ayant une masse molaire faible.

Bien entendu, on peut utiliser au lieu des orifices 27 et 41 des restrictions calibrées quelconques telles que par exemple des capillaires, ou encore des frittés.

Par ailleurs, on prévoit l'utilisation de vannes 29 à membranes qui comportent des moyens de commande de l'actionneur 37 entre les positions de mise en communication et d'isolement, telles que des vannes pneumatiques ou des vannes à commande électromagnétique. Les moyens de commande du déplacement de l'actionneur de la vanne sont alors reliés à une unité de commande, telle que par exemple un micro-ordinateur ou un automate de commande.

II. Dispositif de fourniture d'un gaz pur chargé d'une quantité prédéterminée d'impuretés gazeuses.

On présente ci-après en détail les différentes unités du dispositif de fourniture 9 d'un gaz pur chargé d'une quantité prédéterminée d'impuretés gazeuses, à savoir l'unité d'épuration 17, la source d'impuretés 15 et les moyens 16 de dilution prédéterminée des impuretés dans le gaz pur.

II.1. L'unité d'épuration :

II.1.1. Structure de l'unité d'épuration

L'unité d'épuration 17 comprend une ligne d'entrée 160 et une ligne 162 de fourniture d'un gaz pur, entre lesquelles sont disposés en parallèle trois épurateurs 164, 166, 168, tels que par exemple un épurateur d'azote, un épurateur d'hydrogène et un épurateur d'argon/hélium.

L'entrée de chaque épurateur est raccordée par l'intermédiaire d'une vanne de branchement 170, 172, 174 à la ligne d'entrée 160. La sortie de chaque épurateur 164, 166, 168 est reliée à la ligne de fourniture 162 de l'unité d'épuration 17 au moyen d'une vanne 176, 178, 180 de mise en circuit du gaz épuré.

Les vannes 170 à 180 sont identiques aux vannes 29 des dispositifs de régulation 14A, 14B.

Ainsi, chaque vanne de branchement 170, 172, 174 ainsi que chaque vanne 176, 178, 180 de mise en circuit du gaz épuré comprend un premier conduit 33 relié en permanence par une extrémité à un épurateur correspondant 164, 166, 168. Les deuxièmes conduits 35 des vannes de branchement 170, 172, 174 sont disposés dans la ligne d'entrée 160. Les deuxièmes conduits 35 des vannes 176, 178, 180 de mise en circuit du gaz pur sont disposés dans la ligne de fourniture 162.

La ligne d'alimentation 160 est reliée, en aval de la vanne 174, à une ligne de purge 182 dans laquelle est disposé un organe 184 de création d'une perte de charge, tel qu'un orifice calibré.

La ligne de sortie 162 est reliée, à l'extrémité opposée aux moyens 16 de dilution, à une ligne de purge associée 186 dans laquelle est disposé un organe de création d'une perte de charge 188, tel qu'un orifice calibré.

Les lignes de purge 182 et 186 sont réunies en une ligne de purge commune 190 en aval des orifices calibrés 184 et 188.

II.1.2. Fonctionnement de l'unité d'épuration

En fonction de la sorte de gaz qui est débitée par la source 5, on commute par exemple la vanne d'entrée 170 de l'épurateur 164 associé à ce gaz ainsi que la vanne de sortie correspondante 176 à l'état de mise en communication des conduits 33 et 35. Le gaz à analyser de la source 5 circule librement à travers l'épurateur 164 qui purifie ce gaz. Les autres vannes 172, 174, 178 et 180 sont à l'état d'isolement.

Si on change la sorte de gaz débité par la source de pression 5, on commute les vannes 170, 176 jusqu'alors ouvertes à l'état d'isolement et on va commuter les vannes associées à un autre épurateur à l'état de mise en communication.

Grâce à la construction des vannes 170 à 180 et aux orifices permettant un débit de fuite, les lignes d'alimentation 160 et de sortie 162 sont purgées en permanence. Cette unité d'épuration 23 présente en outre l'avantage qu'elle puisse être utilisée avec différentes sortes de gaz à épurer et qu'elle soit pratiquement libre de volumes de stagnation d'écoulement.

Avantageusement, pour la commutation des vannes, on utilise des vannes à membranes qui comportent des moyens de commande de la commutation de l'actionneur entre les positions de mise en communication et d'isolement, telle que par exemple des vannes pneumatiques ou des vannes à commande électromagnétique. Les moyens de commande du déplacement de l'actionneur de chaque vanne sont reliés à une unité de commande telle que par exemple un micro-ordinateur ou encore un automate logique. Cette unité de commande comporte des moyens logiques de commutation. Ces moyens logiques sont réalisés, par exemple, par un programme informatique chargé dans le micro-ordinateur, qui exclut que deux épurateurs puissent être simultanément en communication avec la ligne d'alimentation 160 et la ligne de sortie 162.

II.2. La source d'impuretés

La source d'impuretés 15 comprend un réservoir contenant un mélange de différentes sortes de gaz telles que par exemple $N_2$, $CO_2$, CO, $O_2$, $CH_4$, $H_2$, Ar, Kr, Xe, He, etc... Ce mélange a été réalisé de telle sorte que les concentrations volumiques de la majorité des gaz de ce mélange sont du même ordre degrandeur (ici le %).

Pour des raisons de sécurité, la concentration d'un gaz inerte, tel que par exemple de l'hélium, est choisie beaucoup plus élevée que la concentration volumique de tous les autres constituants du mélange. Grâce à une telle composition du mélange, des oxydants, tels que $O_2$ ou CO, peuvent coexister ensemble avec des combustibles, tels que par exemple $CH_4$, sans qu'il y ait un risque d'inflammabilité ou d'explosion du réservoir 15.

Afin de connaître précisément, après dilution, le contenu du gaz pur en traces d'impuretés, la composition du mélange du réservoir 15 a été déterminée préalablement avec précision à l'aide de moyens d'analyse des gaz, comme par exemple un chromatographe de phases gazeuses. Le réservoir 15 peut être par exemple une bouteille sous haute pression (typiquement 200 bars).

Les gaz du mélange sont prélevés ou introduits dans les moyens de dilution 16 par l'intermédiaire d'une ligne de prélèvement 204 dans laquelle est placé un régulateur de pression aval 205. La ligne de prélèvement 204 débouche dans une ligne de purge 206 associée dans laquelle sont placés une vanne d'arrêt 207 et un organe de création d'une perte de charge, tel qu'un orifice calibré 208.

II.3. Les moyens de dilution prédéterminée de l'impureté gazeuse dans le gaz pur.

Les moyens de dilution 16 comprennent d'une part les moyens 19 de division du débit du gaz pur délivré par la ligne d'alimentation 162 en sortie de l'unité d'épuration 17, et, d'autre part, trois étages de dilution 20, 21, 22 disposés en série. Une jauge de pression 86 est disposée dans la ligne 162 en amont des moyens de division.

Les moyens de division 19 comprennent le dispositif de régulation 14B et deux lignes 209, 210, reliés en parallèle à la ligne d'alimentation 162. Dans chaque ligne 209, 210 est disposée une restriction calibrée 211, 212 telle qu'un orifice calibré.

La ligne du dispositif de régulation 14B ainsi que les lignes 209 et 210 avec leurs orifices 211, 212 respectifs, forment chacun une branche d'alimentation en gaz pur d'un étage de dilution 20, 21, 22 correspondant.

Le fonctionnement de tels moyens de division de débit est décrit en détail dans la demande de brevet FR-A-2714968 au nom de la Demanderesse. C'est pourquoi ce fonctionnement ne sera pas décrit ci-après.

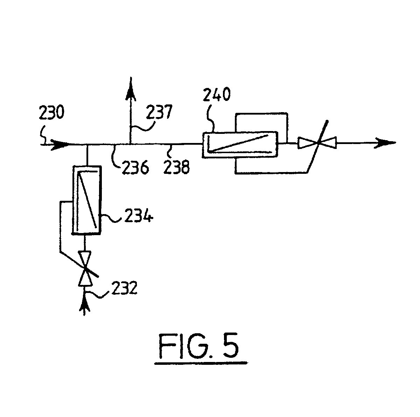

La figure 5 montre un exemple d'un étage de dilution 20, 21 ou 22. Un étage de dilution 20, 21 ou 22 comprend une ligne 230 d'alimentation en gaz pur reliée à une branche correspondante des moyens de division 19, et une ligne 232 d'alimentation en gaz contenant des impuretés.

Un régulateur de débit massique 234 est disposé dans la ligne d'alimentation 232 afin de permettre de faire varier le taux de dilution de l'étage de dilution.

Les lignes d'alimentation 230 et 232 sont raccordées ensemble de telle sorte qu'elles débouchent dans une ligne commune 236 de mélange. La ligne 236 débouche d'une part dans une ligne de sortie 237 de l'étage de dilution. Cette ligne de sortie 237 est reliée à la ligne d'alimentation 232 de l'étage de dilution placé juste en aval.

D'autre part, dans le cas des étages de dilution 20 et 21, la ligne de dilution 236 est reliée à une ligne de purge 238 dans laquelle est disposé un déverseur 240. On règle la pression d'un déverseur dans un étage de dilution de telle sorte que, d'une part, les conditions soniques pour les moyens de dilution sont préférentiellement respectées, et que d'autre part, la pression réglée est un peu supérieure à la pression qui est réglée dans le déverseur placé dans un étage de dilution en aval, afin d'assurer un écoulement des gaz en direction de l'analyseur 3.

La ligne d'alimentation 232 de l'étage de dilution 20 est raccordée, en aval du régulateur de pression 205 et en amont de la vanne d'arrêt 207, à la ligne de prélèvement 204.

La ligne de sortie 237 du dernier étage de dilution 22 débouche dans la ligne de sortie 10 du dispositif de fourniture 9, en aval du dispositif de régulation 14B.

Les débits à l'entrée de chaque étage de dilution sont réglés de telle façon qu'on obtient par exemple dilution voisine de 1/1000 du gaz débité par la ligne 232 dans le gaz pur débité par la ligne 230.

Bien entendu, la quantité "zéro" d'impureté est également une quantité prédéterminée de traces d'impuretés que le dispositif doit fournir à l'analyseur 3. C'est pourquoi la ligne d'alimentation 232 du dernier étage de dilution 22 comporte une ligne de dérivation 242 dans laquelle est placé un régulateur de débit massique 244.

La ligne de sortie 10 du dispositif de fourniture 9 débite un gaz pur dans le cas où on règle le régulateur 244 sur un débit supérieur à celui du régulateur 234 de l'étage de dilution 22, et elle débite un gaz pur chargé d'une quantité prédéterminée de traces d'impuretés dans le cas où le débit réglé par le régulateur 244 est inférieur à celui du régulateur 234.

La génération de l'impureté $H_2O$ est ici réalisée par une cartouche de perméation 250 débitant environ 250ng/mn et reliée en dérivation à la ligne de gaz pur 230 du deuxième étage de dilution 21.

Cette cartouche de perméation contient de l'eau chauffée à 50°C et comporte à une extrémité une membrane de silicone à travers laquelle la molécule $H_2O$ diffuse.

On notera que la cartouche pourrait être placée sur le premier étage pour un taux de perméation plus élevé, le taux de perméation étant choisi pour générer sur la ligne une teneur équivalente à celle des impuretés gazeuses présentes à ce niveau de dilution.

Le tableau 1 ci-dessous montre, pour une composition préalablement déterminée du mélange de gaz dans le réservoir 15, deux exemples de fabrication d'un gaz pur chargé d'une quantité prédéterminée de traces d'impuretés, débité par la ligne de sortie 10 du dispositif de fourniture 9, réalisés une fois avec l'hydrogène comme gaz pur et une fois avec l'azote comme gaz pur.

On conçoit alors que la réserve 15 représente une bouteille unique, comportant toutes les impuretés requises à la calibration de l'appareil (au lieu de multiples mélanges Impureté I/Gaz pur), l'ordre de grandeur initial de la teneur de la majorité des constituants du mélange étant particulièrement confortable à établir, tout en permettant d'obtenir à l'arrivée des impuretés calibrées à des teneurs extrêmement faibles (ppm, ppb, voire plus bas).

Tableau 1

| Impuretés | Composition du réservoir en % volumiques | Concentration des impuretés gaz pur $H_2$ | Concentration des impuretés gaz pur $N_2$ |
|---|---|---|---|
| $O_2$ | 5% | 0,20ppb | 1,62ppb |
| $H_2$ | 5% | - | 1,62ppb |
| $N_2$ | 5% | 0,20ppb | - |
| Ar | 5% | 0,20ppb | 1,62ppb |
| CO | 5% | 0,20ppb | 1,62ppb |
| $CO_2$ | 5% | 0,20ppb | 1,62ppb |
| $CH_4$ | 5% | 0,20ppb | 1,62ppb |
| Xe | 5% | 0,20ppb | 1,62ppb |
| Kr | 5% | 0,20ppb | 1,62ppb |
| He | 55% | 2,19ppb | 17,85ppb |
| $H_2O$ | cartouche de perméation | 0,81ppb | 1,58ppb |

Grâce au fait que la composition initiale du réservoir 15 a été déterminée avec précision au préalable et que ces impuretés sont diluées d'une manière très précise, on obtient un gaz pur contenant une concentration précisément connue des traces d'impuretés.

De plus, à l'aide des débitmètres 234 dans la ligne d'alimentation 232 de chaque étage de dilution 20, 21, 22, on peut faire varier la plage des concentrations des traces d'impuretés d'un facteur 100.

Grâce au fait que le réservoir 15 contient un mélange de plusieurs gaz, celui-ci constitue des traces d'impuretés dans différents gaz purs. Ensemble avec l'unité d'épuration 17, la génération d'un gaz pur chargé de quantités prédéterminées de traces d'impuretés données est alors considérablement facilitée par ce dispositif.

III. Dispositif de sélection d'un de deux gaz :

Comme cela a déjà été décrit, le dispositif de sélection d'un de deux gaz 13 comprend deux lignes d'alimentation 11, 12 dont une 11 est reliée à la ligne d'analyse 7 et l'autre 12 à la ligne de sortie 10 du dispositif de fourniture 9.

Chaque ligne d'alimentation 11, 12 débouche dans une ligne de purge 300 et 302 respective. Les lignes d'alimentation 11 et 12 sont reliées ensemble par l'intermédiaire d'une ligne de raccordement 304. La ligne de raccordement 304 est reliée à l'analyseur 3 par l'intermédiaire d'une ligne de fourniture de gaz 306. La sortie de l'analyseur 3 débouche également dans une ligne de purge 308 associée. Les lignes de purge 300 et 308 comportent chacune un régulateur de débit massique 310, 312. Dans la ligne de purge 302 est disposé un déverseur 314.

Afin de fournir à l'analyseur 3 le gaz contenu dans la ligne 11, on règle la somme des débits $D_{310} + D_{312}$ réglés par les régulateurs de débit 310 et 312 sur un débit inférieur au débit $D_{11}$ débité par la ligne 11. Pour fournir à l'analyseur 3 un gaz d'étalonnage, c'est-à-dire soit un gaz pur, soit un gaz chargé d'une quantité prédéterminée d'impuretés, on règle le débit $D_{310}$ du régulateur de débit 310 pour qu'il soit supérieur au débit $D_{11}$ venant de la ligne 11, et le débit $D_{312}$ réglé par le régulateur de débit 312 pour qu'il soit un peu inférieur au débit de gaz $D_{12}$ de la ligne 12. La pression du gaz introduit dans l'analyseur 3 est réglée par le déverseur 314 disposé dans la ligne de purge 302.

Avantageusement, par un tel agencement, on peut non seulement sélectionner la fourniture à l'analyseur 3 du gaz à analyser ou du gaz d'étalonnage, mais on peut aussi régler des paramètres d'introduction des gaz dans l'analyseur 3 tels que le débit et la pression.

Grâce au fait que l'on utilise un nombre inférieur d'éléments de régulation par rapport au dispositif décrit dans la demande de brevet Français précitée, on diminue considérablement le coût de l'installation. De plus, une diminution du nombre des éléments de régulation entraîne aussi une augmentation de la précision et de la fiabilité de l'appareil 3.

**Revendications**

1. Procédé de fourniture à un appareil (3) d'un gaz pur chargé d'une quantité prédéterminée d'au moins deux impuretés gazeuses, suivant lequel on procède aux mesures suivantes :

   - on dispose d'une source du gaz pur (5, 17);
   - on dispose d'un réservoir où est stocké un mélange de composition connue de gaz auxiliaires, dont lesdites au moins deux impuretés, les concentrations volumiques de la majorité des gaz auxiliaires dans ce mélange étant du même ordre de grandeur, le mélange comprenant au moins un gaz inerte dont la concentration volumique est supérieure à la concentration volumique de chacun des autres gaz du mélange ;
   - on prélève une quantité dudit mélange pour procéder à sa dilution dans le gaz pur, afin d'élaborer par cette dilution ledit gaz pur chargé d'une quantité prédéterminée d'au moins deux impuretés gazeuses,; et
   - on fournit ledit gaz pur ainsi chargé d'une quantité prédéterminée d'au moins deux impuretés gazeuses à l'appareil (3) .

2. Procédé suivant la revendication 1, **caractérisé en ce que** la concentration volumique du gaz inerte est supérieure à la somme des concentrations volumiques des autres gaz du mélange.

3. Procédé suivant l'une des revendications précédentes, **caractérisé en ce que** le dit ordre de grandeur est le %.

4. Dispositif pour la mise en oeuvre du procédé suivant l'une quelconque des revendications 1 à 3, comprenant :

   - une source de gaz pur (5, 17),
   - une source (15) desdites impuretés gazeuses, constituée d'un réservoir contenant un mélange de composition connue de gaz auxiliaires, dont lesdites au moins deux impuretés, les concentrations volumiques de la majorité des gaz auxiliaires dans ce mélange étant du même ordre de grandeur, le mélange comprenant au moins un gaz inerte dont la concentration volumique est supérieure à la concentration volumique de chacun des autres gaz du mélange ;

- des moyens (16) de dilution prédéterminée de l'impureté dans le gaz pur, reliés à l'appareil (3) par l'intermédiaire d'une ligne de fourniture de gaz (10), et aptes à diluer une quantité dudit mélange dans le gaz pur, afin d'élaborer par cette dilution ledit gaz pur chargé d'une quantité prédéterminée d'au moins deux impuretés gazeuses et à fournir ledit gaz pur chargé desdites au moins deux impuretés à l'appareil (3).

5. Dispositif suivant la revendication 4, **caractérisé en ce que** les moyens (16) de dilution comprennent des moyens (19) de division du débit de ladite source (5, 17) de gaz pur, et plusieurs étages de dilution (20, 21, 22) disposés en série, chaque étage (20, 21, 22) comprenant une première ligne d'entrée (230) reliée à une ligne de sortie (85, 209, 210) associée desdits moyens (19) de division de débit et une deuxième ligne d'entrée (232) reliée à une ligne de sortie (237) respective de l'étage de dilution (20, 21) disposé juste en amont, et **en ce que** la deuxième ligne d'entrée (232) du premier étage de dilution (20) est reliée audit réservoir (15) par l'intermédiaire d'une ligne de prélèvement (204) dudit mélange.

6. Dispositif suivant la revendication 5, **caractérisé en ce que** la deuxième ligne d'entrée (232) d'au moins un étage de dilution (20, 21, 22) comporte un organe additionnel (234) de régulation de débit.

7. Dispositif suivant la revendication 5 ou 6, dans lequel chaque étage de dilution (20, 21, 22) comprend des moyens de mélange (236) des gaz alimentant les entrées de l'étage (20, 21, 22), **caractérisé en ce qu'**au moins un étage de dilution (20, 21) comprend une ligne de purge (238) associée, reliée par une extrémité audits moyens de mélange (236) et dans laquelle est disposé un déverseur (240) pour régler la pression dans l'étage de dilution (20, 21) associé.

8. Dispositif suivant l'une quelconque des revendications 5 à 7, **caractérisé en ce qu'**un étage de dilution (21) comprend une troisième ligne d'entrée reliée à des moyens (250) pour charger le gaz pur d'une quantité prédéterminée de $H_2O$.

9. Dispositif suivant l'une quelconque des revendications 5 à 8, **caractérisé en ce que** les moyens (19) de division du débit de ladite source comprennent une ligne principale (162) de fourniture de gaz pur reliée à plusieurs lignes de sortie secondaires (85, 209, 210) disposées en parallèle et comportant chacune une restriction calibrée (27, 211, 212).

10. Dispositif suivant l'une quelconque des revendications 5 à 9, **caractérisé en ce que** la source de gaz pur comprend une source de gaz à analyser (5) et une unité d'épuration (17) du gaz débité par la source (5).

11. Dispositif suivant la revendication 10, **caractérisé en ce que** l'unité d'épuration (17) comprend au moins deux épurateurs (164, 166, 168), l'entrée de chaque épurateur étant reliée à une vanne de branchement (170, 172, 174), disposée dans une ligne d'entrée (160) de l'unité d'épuration (17), et la sortie de chaque épurateur (164, 166, 168) étant reliée à une vanne (176, 178, 180) de mise en circuit du gaz épuré, disposée dans une ligne d'alimentation (162) des moyens de dilution (16).

12. Dispositif suivant la revendication 11, **caractérisé en ce que** la ligne d'entrée (160) de l'unité d'épuration (17) et la ligne d'alimentation (162) des moyens de dilution (16) débouchent chacune par une extrémité dans une ligne de purge (182, 186) correspondante, un organe (184, 188) de création d'une perte de charge étant disposé dans chaque ligne de purge (182, 186) correspondante.

13. Dispositif suivant la revendication 11 ou 12, **caractérisé en ce que** chaque vanne de branchement (170, 172, 174) ainsi que chaque vanne (176, 178, 180) de mise en circuit comprend un premier conduit (33) relié en permanence par une première extrémité à un épurateur (164, 166, 168) associé, un deuxième conduit (35), le deuxième conduit (35) des vannes de branchement étant disposé dans la ligne d'entrée (160) de l'unité d'épuration (17) et celui des vannes de mise en circuit étant disposé dans la ligne d'alimentation (162) des moyens de dilution (16), et un actionneur (37) commutable entre une position de mise en communication du premier conduit (33) avec le deuxième conduit (35) et une position d'isolement du premier conduit (33) par rapport au deuxième conduit (35), le deuxième conduit (35) étant libre de volumes de stagnation d'écoulement.

14. Dispositif selon la revendication 13, **caractérisé en ce que** le deuxième conduit (35) de chaque vanne (170, 172, 174, 176, 178, 180) comporte une chambre (59) dans laquelle débouche la deuxième extrémité du premier conduit (33), et **en ce que** chaque vanne comporte un organe d'obturation (52), sur lequel agit l'actionneur (37) de la vanne, qui obture, dans ladite position d'isolement, l'extrémité du premier conduit (33) débouchant dans ladite chambre (59), et qui est en retrait par rapport à cette extrémité du premier conduit (33) dans ladite position de mise en communication.

15. Dispositif selon la revendication 14, **caractérisé en ce que** l'extrémité du premier conduit (33) débouchant dans ladite chambre (59) est munie d'un joint d'étanchéité (75) en saillie dans la chambre, et **en ce que** l'organe d'obturation (52) comprend une membrane (63) élastiquement déformable formant une partie de paroi de la chambre (59) opposée au joint d'étanchéité (75), la membrane étant appliquée de façon étanche sur le joint d'étanchéité (75) à l'encontre de la force d'élasticité de la membrane, dans ladite position d'isolement, par un poussoir (71) de l'actionneur (37).

16. Dispositif selon l'une quelconque des revendications 13 à 15, **caractérisé en ce que** chaque vanne (170, 172, 174, 176, 178, 180) comporte des moyens de commande de la commutation de l'actionneur (37) entre lesdites positions de mise en communication et d'isolement, **en ce que** les moyens de commande de chaque vanne sont reliés à une unité de commande d'isolement ou de mise en communication de chaque vanne, et **en ce que** l'unité de commande comporte des moyens logiques qui interdisent la commutation simultanée d'actionneurs associés à des épurateurs différents en position de communication.

17. Utilisation d'un réservoir contenant un mélange de composition connue d'au moins trois gaz différents dont deux impureté et un gaz inerte, les concentrations volumiques de la majorité des gaz dudit mélange étant du même ordre de grandeur, et la concentration volumique du gaz inerte étant supérieure à la concentration volumique de chacun des autres gaz du mélange, pour charger un gaz pur avec au moins deux impuretés dans un procédé suivant l'une quelconque des revendications 1 à 3, et/ou pour constituer la source (15) d'impureté d'un dispositif suivant l'une quelconque des revendications 4 à 16.

18. Utilisation suivant la revendication 17, **caractérisée en ce que** la concentration volumique du gaz. inerte est supérieure à la somme des proportions des autres gaz.

## Patentansprüche

1. Verfahren zum Liefern eines reinen Gases, das eine vorbestimmte Menge an mindestens zwei gasförmigen Verunreinigungen enthält, an eine Vorrichtung (3), wobei folgende Schritte durchgeführt werden:

   - eine Quelle an reinem Gas (5, 17) wird zur Verfügung gestellt;

   - ein Behälter wird zur Verfügung gestellt, in dem ein Gemisch in bekannter Zusammensetzung aus Hilfsgasen gelagert ist, unter denen sich die mindestens zwei Verunreinigungen befinden, wobei die Volumenkonzentrationen der Mehrheit der Hilfsgase in diesem Gemisch dieselbe Größenordnung aufweisen, wobei das Gemisch mindestens ein inertes Gas umfasst, dessen Volumenkonzentration größer als die Volumenkonzentration jedes der anderen Gase des Gemisches ist;

   - eine Menge des Gemisches wird entnommen, um sie im reinen Gas zu verdünnen und durch diese Verdünnung das reine Gas zu gewinnen, das eine vorbestimmte Menge an mindestens zwei gasförmigen Verunreinigungen enthält, und

   - das so mit einer vorbestimmten Menge an mindestens zwei gasförmigen Verunreinigungen beaufschlagte reine Gas wird an die Vorrichtung (3) geliefert.

2. Verfahren nach Anspruch 1, **dadurch gekennzeichnet, dass** die Volumenkonzentration des inerten Gases größer als die Summe der Volumenkonzentrationen der anderen Gase des Gemisches ist.

3. Verfahren nach einem der vorhergehenden Ansprüche, **dadurch gekennzeichnet, dass** die Größenordnung Prozent ist.

4. Vorrichtung zur Umsetzung des Verfahrens nach einem der Ansprüche 1 bis 3, umfassend:

- eine Quelle an reinem Gas (5, 17),

- eine Quelle (15) der gasförmigen Verunreinigungen, die aus einem Behälter besteht, der ein Gemisch in bekannter Zusammensetzung aus Hilfsgasen enthält, unter denen sich die mindestens zwei Verunreinigungen befinden, wobei die Volumenkonzentrationen der Mehrheit der Hilfsgase in diesem Gemisch dieselbe Größenordnung aufweisen, wobei das Gemisch mindestens ein inertes Gas umfasst, dessen Volumenkonzentration größer als die Volumenkonzentration jedes der anderen Gase des Gemisches ist;

- Mittel (16) zum vorbestimmten Verdünnen der Verunreinigung im reinen Gas, die mit der Vorrichtung (3) über eine Gaslieferleitung (10) verbunden und dazu geeignet sind, eine Menge des Gemisches im reinen Gas zu verdünnen, um durch diese Verdünnung das reine Gas zu gewinnen, das eine vorbestimmte Menge an mindestens zwei gasförmigen Verunreinigungen enthält, und dazu, das reine Gas, das die mindestens zwei Verunreinigungen enthält, an die Vorrichtung (3) zu liefern.

5. Vorrichtung nach Anspruch 4, **dadurch gekennzeichnet, dass** die Verdünnungsmittel (16) Mittel (19) zum Teilen des Durchsatzes der Quelle (5, 17) an reinem Gas und mehrere Verdünnungsstufen (20, 21, 22), die in Serie angeordnet sind, umfassen, wobei jede Stufe (20, 21, 22) eine erste Eingangsleitung (230), die mit einer Ausgangsleitung (85, 209, 210) verbunden ist, die den Mitteln (19) zum Teilen des Durchsatzes zugeordnet ist, und eine zweite Eingangsleitung (232) umfasst, die mit einer entsprechenden Ausgangsleitung (237) der Verdünnungsstufe (20, 21) verbunden ist, die unmittelbar oberhalb angeordnet ist, und dadurch, dass die zweite Eingangsleitung (232) der ersten Verdünnungsstufe (20) über eine Entnahmeleitung (204) des Gemisches mit dem Behälter (15) verbunden ist.

6. Vorrichtung nach Anspruch 5, **dadurch gekennzeichnet, dass** die zweite Eingangsleitung (232) mindestens einer Verdünnungsstufe (20, 21, 22) ein zusätzliches Element (234) zur Regelung des Durchsatzes umfasst.

7. Vorrichtung nach Anspruch 5 oder 6, wobei jede Verdünnungsstufe (20, 21, 22) Mittel zum Mischen (236) der Gase umfasst, welche die Eingänge der Stufe (20, 21, 22) versorgen, **dadurch gekennzeichnet, dass** mindestens eine Verdünnungsstufe (20, 21) eine dazugehörige Spülleitung (238) umfasst, die über ein Ende mit den Mitteln zum Mischen (236) verbunden ist und in der ein Auslauf (240) angeordnet ist, um den Druck in der dazugehörigen Verdünnungsstufe (20, 21) zu regeln.

8. Vorrichtung nach einem der Ansprüche 5 bis 7, **dadurch gekennzeichnet, dass** eine Verdünnungsstufe (21) eine dritte Eingangsleitung umfasst, die mit Mitteln (250) zum Beaufschlagen des reinen Gases mit einer vorbestimmten Menge an $H_2O$ verbunden ist.

9. Vorrichtung nach einem der Ansprüche 5 bis 8, **dadurch gekennzeichnet, dass** die Mittel (19) zum Teilen des Durchsatzes der Quelle eine Hauptleitung (162) zum Liefern des reinen Gases umfassen, die mit mehreren sekundären Ausgangsleitungen (85, 209, 210) verbunden ist, die parallel angeordnet sind und von denen jede eine kalibrierte Drossel (27, 211, 212) aufweist.

10. Vorrichtung nach einem der Ansprüche 5 bis 9, **dadurch gekennzeichnet, dass** die Quelle an reinem Gas eine Quelle (5) an zu analysierendem Gas und eine Reinigungseinheit (17) des von der Quelle (5) durchgesetzten Gases umfasst.

11. Vorrichtung nach Anspruch 10, **dadurch gekennzeichnet, dass** die Reinigungseinheit (17) mindestens zwei Reinigungsvorrichtungen (164, 166, 168) umfasst, wobei der Eingang jeder Reinigungsvorrichtung mit einem Abzweigventil (170, 172, 174) verbunden ist, das in einer Eingangsleitung (160) der Reinigungseinheit (17) angeordnet ist, und wobei der Ausgang jeder Reinigungsvorrichtung (164, 166, 168) mit einem Ventil (176, 178, 180) zum Zirkulieren des gereinigten Gases verbunden ist, das in einer Versorgungsleitung (162) der Verdünnungsmittel (16) angeordnet ist.

12. Vorrichtung nach Anspruch 11, **dadurch gekennzeichnet, dass** die Eingangsleitung (160) der Reinigungseinheit (17) und die Versorgungsleitung (162) der Verdünnungsmittel (16) jeweils mit einem Ende in eine entsprechende Spülleitung (182, 186) münden, wobei ein Element (184, 188) zum Erzeugen eines Beaufschlagungsverlustes in jeder entsprechenden Spülleitung (182, 186) angeordnet ist.

13. Vorrichtung nach Anspruch 11 oder 12, **dadurch gekennzeichnet, dass** jedes Abzweigventil (170, 172, 174) sowie jedes Zirkulationsventil (176, 178, 180) eine erste Leitung (33), die ständig über ein erstes Ende mit einer dazugehörigen Reinigungsvorrichtung (164, 166, 168) verbunden ist, eine zweite Leitung (35), wobei die zweite Leitung (35) der Abzweigventile in der Eingangsleitung (160) der Reinigungseinheit (17) und jene der Zirkulationsventile in der Versorgungsleitung (162) der Verdünnungsmittel (16) angeordnet ist, und ein Stellglied (37) umfasst, das zwischen einer Stellung zum Verbinden der ersten Leitung (33) mit der zweiten Leitung (35) und einer Stellung zum Isolieren der ersten Leitung (33) von der zweiten Leitung (35) schaltbar ist, wobei die zweite Leitung (35) frei von Abflussstagnationsvolumina ist.

14. Vorrichtung nach Anspruch 13, **dadurch gekennzeichnet, dass** die zweite Leitung (35) jedes Ventils (170, 172, 174, 176, 178, 180) eine Kammer (59) aufweist, in die das zweite Ende der ersten Leitung (33) mündet, und dadurch, dass jedes Ventil ein Verschlusselement (52), auf welches das Stellglied (37) des Ventils wirkt, aufweist, das in der Isolierungsposition das Ende der ersten Leitung (33), das in die Kammer (59) mündet, verschließt und in der Verbindungsstellung in Bezug auf dieses Ende der ersten Leitung (33) zurückgezogen ist.

15. Vorrichtung nach Anspruch 14, **dadurch gekennzeichnet, dass** das Ende der ersten Leitung (33), das in die Kammer (59) mündet, mit einer Dichtung (75) versehen ist, die in der Kammer hervorsteht, und dadurch, dass das Verschlusselement (52) eine elastisch verformbare Membran (63) umfasst, die einen Teil der Wand der Kammer (59) bildet, die der Dichtung (75) gegenüber liegt, wobei die Membran in der Isolierungsstellung durch einen Tastschalter (71) des Stellglieds (37) dicht an der Dichtung (75) gegen die Elastizitätskraft der Membran angebracht wird.

16. Vorrichtung nach einem der Ansprüche 13 bis 15, **dadurch gekennzeichnet, dass** jedes Ventil (170, 172, 174, 176, 178, 180) Steuermittel zum Schalten des Stellglieds (37) zwischen der Verbindungsstellung und der Isolierungsstellung aufweist, dadurch, dass die Steuermittel jedes Ventils an eine Steuereinheit zum Isolieren oder Verbinden jedes Ventils angeschlossen sind, und dadurch, dass die Steuereinheit logische Mittel aufweist, die in der Verbindungsstellung das gleichzeitige Schalten von Stellgliedern, die unterschiedlichen Reinigungsvorrichtungen zugeordnet sind, verhindern.

17. Verwendung eines Behälters, der ein Gemisch in bekannter Zusammensetzung aus mindestens drei unterschiedlichen Gasen enthält, unter denen sich zwei Verunreinigungen und ein inertes Gas befinden, wobei die Volumenkonzentrationen der Mehrheit der Gase des Gemisches dieselbe Größenordnung aufweisen und die Volumenkonzentration des inerten Gases größer als die Volumenkonzentration jedes der anderen Gase des Gemisches ist, um ein reines Gas in einem Verfahren nach einem der Ansprüche 1 bis 3 mit mindestens zwei Verunreinigungen zu beaufschlagen und/oder um die Quelle (15) an Verunreinigungen einer Vorrichtung nach einem der Ansprüche 4 bis 16 darzustellen.

18. Verwendung nach Anspruch 17, **dadurch gekennzeichnet, dass** die Volumenkonzentration des inerten Gases größer als die Summe der Proportionen der anderen Gase ist.

**Claims**

1. Method of delivering a pure gas charged with a predetermined quantity of at least two gaseous impurities to an apparatus (3), in which the following measures are taken:

 - a source (5, 17) of pure gas is used;
 - a reservoir is used in which a mixture of known composition of auxiliary gases, including the said at least two impurities, is stored, the volume concentrations of the majority of the auxiliary gases in this mixture being of the same order of magnitude, the mixture comprising at least one inert gas whose volume concentration is greater than the volume concentration of each of the other gases of the mixture;
 - a quantity of the said mixture is taken off in order to dilute it in the pure gas, so as to produce, by this dilution, the said pure gas charged with a predetermined quantity of at least two gaseous impurities; and
 - the said pure gas thus charged with a predetermined quantity of at least two gaseous impurities is delivered to the apparatus (3).

2. Method according to Claim 1, **characterized in that** the volume concentration of the inert gas is greater than the sum of the volume concentrations of the other gases of the mixture.

**3.** Method according to either of the preceding claims, **characterized in that** the said order of magnitude is 1%.

**4.** Plant for implementing the method according to any one of Claims 1 to 3, comprising:

- a source (5, 17) of pure gas;
- a source (15) of the said gaseous impurities, consisting of a reservoir containing a mixture of known composition of auxiliary gases, including the said at least two impurities, the volume concentrations of the majority of the auxiliary gases in this mixture being of the same order of magnitude, the mixture comprising at least one inert gas whose volume concentration is greater than the volume concentration of each of the other gases of the mixture;
- means (16) for the predetermined dilution of the impurity in the pure gas, which means are connected to the apparatus (3) via a gas delivery line (10) and are capable of diluting a quantity of the said mixture in the pure gas, so as to produce, by this dilution, the said pure gas charged with a predetermined quantity of at least two gaseous impurities and to deliver the said pure gas charged with the said at least two impurities to the apparatus (3).

**5.** Plant according to Claim 4, **characterized in that** the dilution means (16) comprise means (19) for dividing the stream from the said source (5, 17) of pure gas and several dilution stages (20, 21, 22) arranged in series, each stage (20, 21, 22) comprising a first incoming line (230) connected to an associated outgoing line (85, 209, 210) of the said flow dividing means (19) and a second incoming line (232) connected to a respective outgoing line (237) of the dilution stage (20, 21) placed just upstream, and **in that** the second incoming line (232) of the first dilution stage (20) is connected to the said reservoir (15) via a line (204) for taking off the said mixture.

**6.** Plant according to Claim 5, **characterized in that** the second incoming line (232) of at least one dilution stage (20, 21, 22) includes an additional flow regulator (234).

**7.** Plant according to Claim 5 or 6, in which each dilution stage (20, 21, 22) comprises means (236) for mixing the gases feeding the inlets of the stages (20, 21, 22), **characterized in that** at least one dilution stage (20, 21) includes an associated purge line (238) connected via one end to the said mixing means (236) and in which a diverter (240) is placed in order to regulate the pressure in the associated dilution stage (20, 21).

**8.** Plant according to any one of Claims 5 to 7, **characterized in that** a dilution stage (21) includes a third incoming line connected to means (250) for charging the pure gas with a predetermined quantity of $H_2O$.

**9.** Plant according to any one of Claims 5 to 8, **characterized in that** the means (19) for dividing the stream from the said source comprise a main line (162) for delivering pure gas connected to several secondary outgoing lines (85, 209, 210) arranged in parallel, each having a calibrated restriction (27, 211, 212).

**10.** Plant according to any one of Claims 5 to 9, **characterized in that** the source of pure gas comprises a source (5) of gas to be analysed and a unit (17) for purifying the gas output by the source (5).

**11.** Plant according to Claim 10, **characterized in that** the purifying device (17) comprises at least two purifiers (164, 166, 168), the inlet of each purifier being connected to a branching valve (170, 172, 174) placed in an incoming line (160) for the purifying unit (17), and the outlet of each purifier (164, 166, 168) being connected to a purified-gas injection valve (176, 178, 180) placed in a supply line (162) for the dilution means (16).

**12.** Plant according to Claim 11, **characterized in that** the incoming line (160) of the purification unit (17) and the supply line (162) of the dilution means (16) each run via one end into a corresponding purge line (182, 186), a member (184, 188) for creating a head loss being placed in each corresponding purge line (182, 186).

**13.** Plant according to Claim 11 or 12, **characterized in that** each branching valve (170, 172, 174) and each injection valve (176, 178, 180) includes a first conduit (33) permanently connected via a first end to an associated purifier (164, 166, 168), a second conduit (35), the second conduit (35) of the branching valves being placed in the incoming line (160) of the purification unit (17) and that of the injection valves being placed in the supply line (162) of the dilution means (16), and an actuator (37) which can switch between a position for bringing the first conduit (33) into communication with the second conduit (35) and a position for isolating the first conduit (33) from the second conduit (35), the second conduit (35) being free of flow stagnation volumes.

**14.** Plant according to Claim 13, **characterized in that** the second conduit (35) of each valve (170, 172, 174, 176, 178, 180) includes a chamber (59) into which the second end of the first conduit (33) runs and **in that** each valve includes a closure member (52) which is acted upon by the actuator (37) of the valve, which, in the said isolating position, closes off the end of the first conduit (33) running into the said chamber (59) and is set back with respect to this end of the first conduit (33) in the said communicating position.

**15.** Plant according to Claim 14, **characterized in that** the end of the first conduit (33) running into the said chamber (59) is provided with a seal (75) projecting into the chamber and **in that** the closure element (52) comprises an elastically deformable diaphragm (63) forming part of the wall of the chamber (59) opposite the seal (75), the diaphragm being pressed in a sealed manner onto the seal (75) against the elastic force of the diaphragm, in the said isolating position, by a pusher (71) of the actuator (37).

**16.** Plant according to any one of Claims 13 to 15, **characterized in that** each valve (170, 172, 174, 176, 178, 180) includes means for making the actuator (37) switch between the said communicating position and the isolating position, **in that** the means for operating each valve are connected to a control unit for the isolating or communicating function of each valve and **in that** the control unit includes logic means which prevent the simultaneous switching of actuators associated with different purifiers into the communication position.

**17.** Use of a reservoir containing a mixture of known composition of at least three different gases, including two impurities and an inert gas, the volume concentrations of the majority of the gases of the said mixture being of the same order of magnitude and the volume concentration of the inert gas being greater than the volume concentration of each of the other gases of the mixture, in order to charge a pure gas with at least two impurities in a method according to any one of Claims 1 to 3 and/or to constitute the source (15) of impurity of a plant according to any one of Claims 4 to 16.

**18.** Use according to Claim 17, **characterized in that** the volume concentration of the inert gas is greater than the sum of the proportions of the other gases.

FIG.1

FIG.3

FIG.2

FIG.4A

FIG. 4B

## FIG. 5